# EUROPEAN PATENT APPLICATION

(11) **EP 4 491 958 A1**
(43) Date of publication of application: **15.01.2025**
(21) Application number: 22930792.1
(22) Date of filing: 09.03.2022
(51) Int. Cl.: F24F 7/06

(54) **CLEAN ROOM FACILITY**

(71) Applicant: Hitachi Global Life Solutions, Inc., Tokyo 105-8410 (JP)
(72) Inventor: NISHIMURA, Noritoshi, Tokyo 105-8410 (JP); MATSUZAKI, Kazuhito, Tokyo 105-8410 (JP); IMAGUCHI, Nobuhiro, Tokyo 105-8410 (JP)
(74) Representative: MERH-IP Matias Erny Reichl Hoffmann Patentanwälte PartG mbB
(86) International application number: PCT/JP2022/010208
(87) International publication number: WO 2023/170808

(57) **Abstract**

A clean room facility that improves efficiency of sample preparation is provided. The clean room facility (100) includes a processing room (R3) in which a safety cabinet (32) used for preparing a sample is arranged, a plurality of rooms (R6, R7, R8) in which incubators (41 to 43) related to culture or inspection of the sample are arranged, a partition (6a, 7a, 8a) that partitions the processing room (R3) and each of the rooms (R6, R7, R8), an openable and closable door (6b, 7b, 8b) or a window that is provided in the partition (6a, 7a, 8a) and used for moving of the sample, and a side wall (6d, 7d) that partitions adjacent rooms among the plurality of rooms (R6, R7, R8).

## Description

### Technical Field

The present invention relates to a clean room facility.

### Background Art

A clean room having high air cleanliness is used in regenerative medicine, production of pharmaceutical products, and the like. In relation to such a clean room, for example, PTL 1 discloses a configuration of "a unit type cell culture facility installed in a building, which includes an indoor partition portion that partitions the building into a plurality of rooms, a ceiling embedded air conditioner that takes in outside air, and an in-ceiling chamber in which air is supplied from the ceiling embedded air conditioner". PTL 1 discloses that a safety cabinet for handling cultured cells is installed in an operation room for performing work related to cell culture, and in addition, it is preferable to install an incubator or the like related to cell culture.

### Citation List

### Patent Literature

PTL 1: JP2013-240358A

### Summary of Invention

### Technical Problem

In the technique disclosed in PTL 1, the operation room is a room for cell culture dedicated to a patient. Therefore, when cell culture of a different patient is performed after cell culture of a patient in the incubator in the operation room is completed, sterilization in the operation room is often performed. However, the technique disclosed in PTL 1 does not consider efficiency of sample preparation related to cell culture or the like.

Therefore, an object of the invention is to provide a clean room facility that improves efficiency of sample preparation.

### Solution to Problem

In order to solve the problem described above, a clean room facility according to the invention includes a processing room configured to allow a first device used for preparing a sample to be arranged therein, a plurality of rooms configured to allow second devices related to culture or inspection of the sample to be arranged therein, a partition partitioning the processing room and each of the rooms, an openable and closable door or window provided in the partition and used for moving of the sample, and a wall partitioning adjacent rooms among the plurality of rooms.

### Advantageous Effects of Invention

According to the invention, it is possible to provide a clean room facility that improves efficiency of sample preparation.

### Brief Description of Drawings

[FIG. 1] FIG. 1 is a diagram showing a layout of rooms of a clean room facility according to a first embodiment.
[FIG. 2] FIG. 2 is a schematic cross-sectional view showing the clean room facility according to the first embodiment.
[FIG. 3] FIG. 3 is a configuration diagram related to control of a fan filter unit on an air supply side and a fan filter unit on an air returning side in the clean room facility according to the first embodiment.
[FIG. 4] FIG. 4 is a diagram showing an example related to treatment during sterilization of a room in the clean room facility according to the first embodiment.
[FIG. 5] FIG. 5 is a diagram showing another example related to treatment during sterilization of a room in the clean room facility according to the first embodiment.
[FIG. 6] FIG. 6 is a diagram showing a layout of rooms in a clean room facility according to a second embodiment.
[FIG. 7] FIG. 7 is a diagram including a conveyance machine of the clean room facility according to the second embodiment.
[FIG. 8] FIG. 8 is a schematic cross-sectional view showing a clean room facility according to a first modification.
[FIG. 9] FIG. 9 is a schematic cross-sectional view showing a clean room facility according to a second modification.
[FIG. 10] FIG. 10 is a diagram showing a layout of rooms in a clean room facility according to a comparative example.

### Description of Embodiments

### <<First Embodiment>>

### <Configuration of Clean Room Facility>

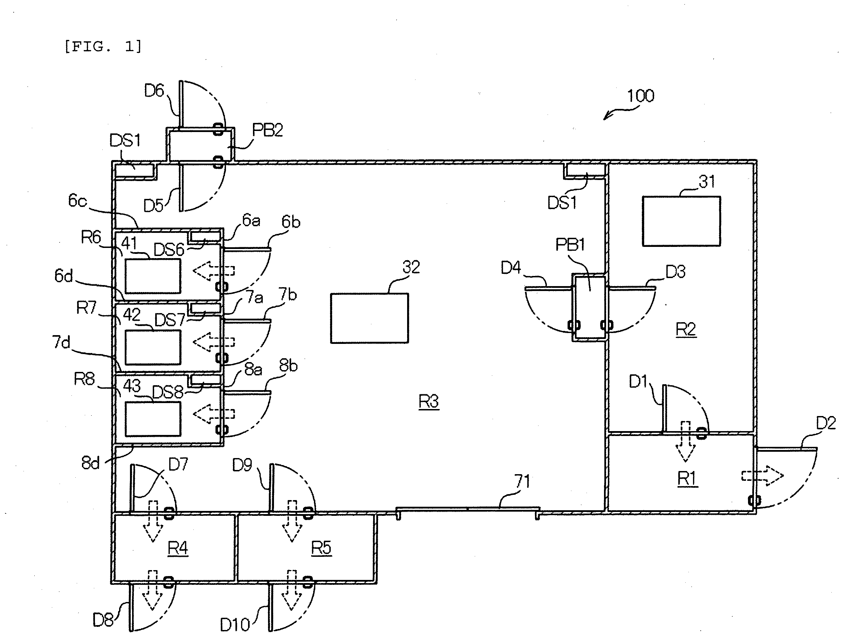

FIG. 1 is a diagram showing a layout of rooms of a clean room facility 100 according to a first embodiment.

In FIG. 1, a direction in which air flows when a predetermined door (for example, a door D1) is opened is indicated by a dashed white arrow. The clean room facility 100 is a facility for adjusting a temperature, room pressure, cleanliness, and the like of a plurality of clean rooms such as a pretreatment room R2 and a processing room R3, and for example, the clean room facility 100 is used for culture processing of cells and production of a sterile preparation (vaccine, injection, eye drop, and the like). The clean room facility 100 shown in FIG. 1 is provided with a changing room R1, the pretreatment room R2, the processing room R3, a dressing room R4, an undressing room R5, and three rooms R6, R7, and R8 as clean rooms.

In such a clean room facility 100, a plurality of clean rooms having different degrees of air cleanliness are often provided. In order to prevent air leakage from a clean room with low cleanliness to a clean room with high cleanliness, a room pressure difference is provided between adjacent clean rooms. For example, the processing room R3 shown in FIG. 1 has higher air cleanliness and higher room pressure than the dressing room R4. Therefore, in a case where an operator opens a door D7 when the operator enters the processing room R3 from the dressing room R4, air flows from the processing room R3 having high pressure to the dressing room R4 having low pressure as indicated by a dashed arrow in FIG. 1, but the reverse flow hardly occurs. Accordingly, entrance of aerosol and dust from the dressing room R4 into the processing room R3 is prevented, and the processing room R3 is kept clean. Cleanliness of the dressing room R4 may be equal to cleanliness of the processing room R3.

In FIG. 1, when there is a dashed white arrow directed from one clean room of two adjacent clean rooms to the other clean room, it is assumed that room pressure of the one clean room is higher than room pressure of the other clean room. The changing room R1 shown in FIG. 1 is a room for an operator to change clothes before and after work in the pretreatment room R2. An operator can move back and forth between the changing room R1 and the pretreatment room R2 through the door D1.

The pretreatment room R2 is a clean room in which an operator pre-treats a sample. The pretreatment room R2 is provided with a safety cabinet 31 for handling a sample. A pass box PB1 shown in FIG. 1 is a box with double doors used when a sample is moved between the pretreatment room R2 and the processing room R3. Sample contamination can be prevented by moving the sample using such a pass box PB1. Although a door through which a person can enter and exit is not particularly provided between the pretreatment room R2 and the processing room R3 in the example shown in FIG. 1, such a door can be provided as appropriate.

The processing room R3 is a clean room in which a sample is prepared. Here, "a sample is prepared" refers to performing a predetermined operation or processing on the sample. Examples of the "sample" include, but are not limited to, cells and sterile preparations. Cleanliness of the processing room R3 is higher than cleanliness of the pretreatment room R2. Room pressure of the processing room R3 is higher than room pressure of the pretreatment room R2. The processing room R3 may be provided with an equipment carry-in door 71 which is opened and closed when predetermined equipment is carried in.

As shown in FIG. 1, a safety cabinet 32 (a first device) used to prepare a sample is disposed in the processing room R3. The safety cabinet 32 is a box-shaped device configured such that an operator can treat a sample in a clean environment in which contamination of foreign substances, microorganisms, and the like is prevented. The safety cabinet 32 includes, for example, a housing (not shown) having front and side surfaces formed of transparent glass or resin, and a work area in the housing can be visually recognized from the outside.

An opening (not shown) for an operator to take in and out a hand is provided in the front surface of the housing (not shown) of the safety cabinet 32. As described above, the safety cabinet 32 is a device of an open type in which a work area in the housing communicates with an outside space through the opening. A sample treated in the safety cabinet 32 is carried out via a pass box PB2.

Instead of the safety cabinet 31 in the processing room R3, an isolator (the first device: not shown) may be used to prepare a sample. The isolator includes a housing (not shown) having front and side surfaces formed of transparent glass or resin, and a work area in the housing can be visually recognized from the outside. A sample can be handled in a state in which an operator puts a hand into a glove (not shown) installed in the front surface of the housing. Such an isolator is a so-called closed type device, and a work area is closed. Pressure inside the housing of the isolator may be positive or negative relative to room pressure of the processing room R3.

In the example shown in FIG. 1, the dressing room R4 and the undressing room R5 are provided adjacent to the processing room R3. The dressing room R4 is a room for an operator to change clothes to predetermined clean room wear. The operator who changes clothes to the clean room wear in the dressing room R4 opens the door D7 and enters the processing room R3. The undressing room R5 is a room in which the operator takes off the clean room wear and changes clothes to predetermined clothes. The operator who finished work in the processing room R3 opens a door D9 and enters the undressing room R5.

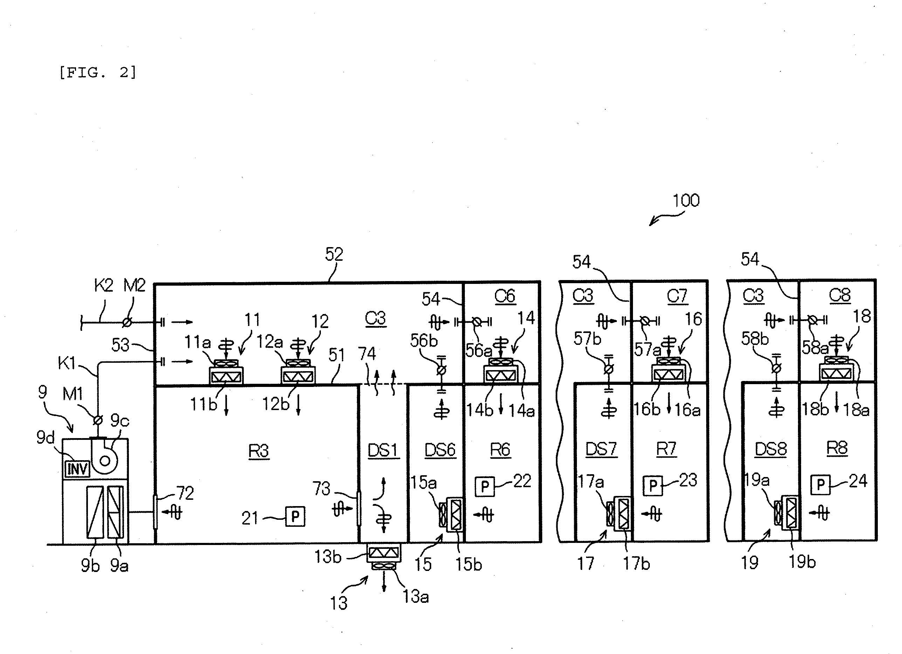

A duct shaft DS1 shown in FIG. 1 is an air guide pipe that guides a part of air flowing out from the processing room R3 to a chamber C3 (see FIG. 2) behind a ceiling. Although not shown in FIG. 1, a duct shaft is also appropriately provided in other clean rooms such as the pretreatment room R2.

In the example shown in FIG. 1, three rooms R6, R7, and R8 are sequentially provided adjacent to each other. The rooms R6, R7, and R8 are spaces in which devices (second devices) related to sample culture or inspection are arranged. In the example shown in FIG. 1, an incubator 41 (the second device) for culturing a sample is provided in the room R6. Similarly, an incubator 42 (the second device) is provided in another room R7, and an incubator 43 (the second device) is provided in the remaining room R8.

The incubator 41 provided in the room R6 is a device for culturing a sample as described above. The incubator 41 has a box shape, and an inner side is adjusted to a temperature and humidity suitable for culturing a sample. The incubator 41 as described above includes a housing (not shown) and a door (not shown) installed in the housing. The housing is provided with a plurality of shelves (not shown) for placing a container in which a sample is placed. In addition to the temperature and humidity of the incubator 41, a carbon dioxide concentration or the like may be appropriately adjusted. The other incubators 42 and 43 also have the same configuration.

As shown in FIG. 1, the room R6 includes a partition 6a, a door 6b, and a pair of side walls 6c and 6d. The partition 6c is a plate (or a wall) that partitions the processing room R3 and the room R6, and extends from a floor surface to a ceiling of the room R6. The door 6b is an openable and closable door used for moving a sample, and is provided in the partition 6a. The pair of side walls 6c and 6d are walls that form a right side and a left side of the room R6 when viewed from a person facing the door 6b. The side wall 6c (one) faces a wall of the processing room R3. The side wall 6d (the other one) is a "wall" that partitions the adjacent rooms R6 and R7 among the plurality of rooms R6, R7, and R8. The pair of side walls 6c and 6d both extend from the floor surface to the ceiling of the room R6. In the example shown in FIG. 1, in the room R6, a wall opposite to the partition 6a provided with the door 6b is substantially formed integrally with a wall of the processing room R3.

When the door 6b shown in FIG. 1 is closed, the room R6 is separated from the processing room R3 and the other rooms R7 and R8. Accordingly, even when sample contamination occurs during work in the room R6, it is possible to prevent sample contamination from reaching the processing room R3 and the other rooms R7 and R8. Since it is not particularly necessary to sterilize the processing room R3 and the other rooms R7 and R8 when the room R6 is sterilized, it is possible to reduce labor and cost required for sterilization.

As shown in FIG. 1, a duct shaft DS6 is provided in the room R6. The duct shaft DS6 is an air guide pipe that guides air flowing out from the room R6 to the chamber C3 (see FIG. 2) behind a ceiling of the processing room R3. The other rooms R7 and R8 also have the same configuration as that of the room R6. The partition 6a of the room R6, a partition 7a of the room R7, and a partition 8a of the room R8 may be integrally formed.

In the central room R7 among the three rooms R6, R7, and R8, side walls 6d and 7d function as "walls" that partition the room R7 from the other rooms. That is, the side wall 6d functions as a "wall" for partitioning the rooms R6 and R7. Similarly, the side wall 7d functions as a "wall" for partitioning the rooms R7 and R8.

Cleanliness of the processing room R3 may be equal to cleanliness of each of the rooms R6, R7, and R8. As the number of times of ventilation per unit time of the processing room R3 or the like increases, cleanliness increases. Room pressure of the processing room R3 may be different from room pressure of each of the plurality of rooms R6, R7, and R8. In particular, the room pressure of the processing room R3 is preferably higher than the room pressure of each of the plurality of rooms R6, R7, and R8. Accordingly, for example, even when sample contamination occurs in the room R6, it is possible to prevent dust and aerosol from leaking from the room R6 to the processing room R3 through minute gaps of the door 6b. Cleanliness and set pressure of the rooms R6, R7, and R8 may be equal to or different from one another.

FIG. 2 is a schematic cross-sectional view showing the clean room facility 100.

In FIG. 2, the processing room R3 and the three rooms R6, R7, and R8 are shown, and the pretreatment room R2 (see FIG. 1) and the like are omitted as appropriate. In FIG. 2, an air flow is indicated by a solid arrow. FIG. 2 is a schematic cross-sectional view focusing on an air flow such as an air flow of guiding air from the processing room R3 to the chamber C3 through the duct shaft DS1. In FIG. 2, the safety cabinet 32 (see FIG. 1) and the incubators 41 to 43 (see FIG. 1) are omitted.

As shown in FIG. 2, the clean room facility 100 includes an air conditioner 9, fan filter units 11 to 19, and pressure sensors 21 to 24. The air conditioner 9 is a device that adjusts a temperature or the like of air, and includes a filter 9a, a cooling coil 9b, and a fan 9c. The air conditioner 9 may include an inverter 9d.

The filter 9a captures dust from air flowing from the processing room R3 toward the cooling coil 9b via a predetermined gap 61. The cooling coil 9b is a heat exchanger that exchanges heat between air passed through the filter 9a and a refrigerant flowing through a heat transfer pipe (not shown). The fan 9c is a blower that pumps air whose temperature or the like is adjusted by the cooling coil 9b to the chamber C3 via a duct K1. The inverter 9d is a power converter that drives a motor (not shown) of the fan 9c.

In the example shown in FIG. 2, the air whose temperature or the like is adjusted is guided to the chamber C3 through another duct K2 in addition to the duct K1. A damper M1 adjusts a flow rate of air, and is provided in the duct K1. Similarly, another damper M2 is provided in the duct K2. During a general operation of the clean room facility 100, opening degrees of the dampers M1 and M2 are maintained at predetermined values.

The chamber C3 shown in FIG. 2 is a space behind a ceiling of the processing room R3. The chamber C3 includes a ceiling 51 of the processing room R3, an upper plate 52 at a position higher than the ceiling 51, a side plate 53, and a separation wall 54. The side plate 53 is installed on edge portions on one side in a lateral direction of the ceiling 51 and the upper plate 52. The separation wall 54 is a wall that partitions the processing room R3 from the rooms R6, R7, and R8, and is installed on edge portions on the other side in the lateral direction of the ceiling 51 and the upper plate 52. The space behind the ceiling of the processing room R3 or a space behind a ceiling of the pretreatment room R2 or the like (see FIG. 1) may be formed as one chamber.

The fan filter units 11 and 12 shown in FIG. 2 are devices that supply air from the chamber C3 to the processing room R3, and are embedded in the ceiling 51 of processing room R3. The fan filter unit 11 includes an air supply fan 11a and a filter 11b. The air supply fan 11a is a blower that supplies air from the chamber C3 to the processing room R3. The filter 11b captures dust from the air flowing from the air supply fan 11a to the processing room R3, and is provided on an air outlet side of the air supply fan 11a. Examples of the filter 11b include high efficiency particulate air filter (HEPA) and ultra low penetration air filter (ULPA). The fan filter unit 12 also has the same configuration.

The fan filter unit 13 shown in FIG. 2 is a device that performs exhaustion and air returning from the processing room R3, and includes an air returning fan 13a and a filter 13b. The "air returning" from the processing room R3 refers to that at least a part of air flowing out from the processing room R3 is returned to the processing room R3 through the duct shaft DS1 and the like. In the example shown in FIG. 2, when the air returning fan 13a is driven, air is guided from the processing room R3 to the duct shaft DS1 through a predetermined gap 73. A part of the air guided to the duct shaft DS1 is returned to the chamber C3 through the duct shaft DS1, and the remaining air is exhausted. A porous plate 74 (or grating) is provided at a downstream end of the duct shaft DS1.

Although the fan filter unit 13 is shown on a lower side of a paper surface of a floor of the processing room R3 for simplification in FIG. 2, the fan filter unit 13 is embedded in a side wall of the processing room R3 in practice. The pressure sensor 21 shown in FIG. 2 is a sensor that detects room pressure of the processing room R3, and is provided in the processing room R3. The air supply fans 11a and 12a and the air returning fan 13a are controlled such that the room pressure of the processing room R3 is predetermined set pressure (target pressure).

The clean room facility 100 includes a room chamber C6, the fan filter units 14 and 15, the pressure sensor 22, the duct shaft DS6, a first damper 56a, and a second damper 56b as a configuration corresponding to the room R6 (see FIG. 1).

The room chamber C6 is a space behind a ceiling of the room R6. The room chamber C6 is partitioned from an adjacent room chamber C7 by a wall member (not shown), and is partitioned from the chamber C3 behind the ceiling of the processing room R3 by the separation wall 54. The same applies to the room chamber C7 behind a ceiling of the room R7 and a room chamber C8 behind a ceiling of the other room R8. In this manner, the three room chambers C6, C7, and C8 behind ceilings are individually provided to correspond to the respective three rooms R6, R7, and R8.

The fan filter unit 14 shown in FIG. 2 includes an air supply fan 14a and a filter 14b, and is embedded in the ceiling of the room R6. The air supply fan 14a is a blower that supplies air to the room R6 (a room corresponding to the room chamber C6), and is provided in the room chamber C6. The filter 14b captures dust from air flowing from the room chamber C6 to the room R6, and is provided on an air outlet side of the air supply fan 14a.

The fan filter unit 15 shown in FIG. 2 includes an air returning fan 15a and a filter 15b, and is embedded in a side wall of the room R6. The air returning fan 15a is a blower that returns air flowing out from the room R6 to the chamber C3 behind the ceiling of the processing room R3 through the duct shaft DS6. The filter 15b captures dust from air suctioned into the air returning fan 15a from the room R6, and is provided on a suction side of the air returning fan 15a.

The pressure sensor 22 shown in FIG. 2 is a sensor that detects the room pressure of the room R6, and is provided in the room R6. The air supply fan 14a and the air returning fan 15a are controlled such that the room pressure of the room R6 is predetermined set pressure (target pressure).

The duct shaft DS6 is an air guide pipe that guides air flowing out from the room R6 to the chamber C3 behind the ceiling of the processing room R3. The porous plate 74 (or grating) is provided at a downstream end of the duct shaft DS6. The first damper 56a shown in FIG. 2 enables or blocks communication between the room chamber C6 and the chamber C3 behind the ceiling of the processing room R3, and is provided in the separation wall 54 of the room chamber C6 corresponding to the room R6.

The second damper 56b enables or blocks communication between the duct shaft DS6 and the chamber C3, and is provided near the downstream end of the duct shaft DS6 to correspond to the room R6. Configurations corresponding to the remaining two rooms R7 and R8 are the same as those described above corresponding to the room R6.

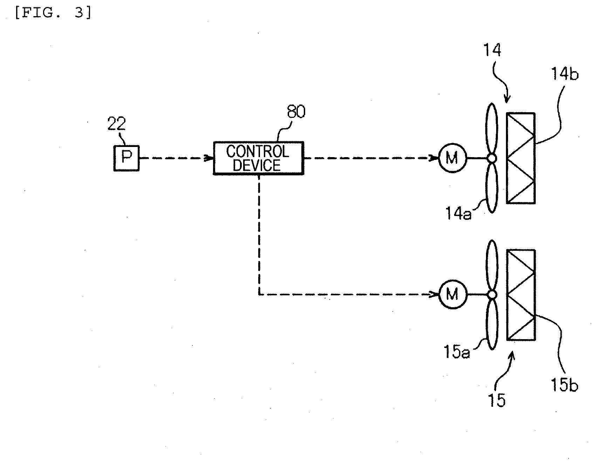

FIG. 3 is a configuration diagram related to control of the fan filter unit 14 on an air supply side and the fan filter unit 15 on an air returning side.

The clean room facility 100 (see FIG. 2) has a configuration corresponding to the room R6 (see FIG. 2), and includes a control device 80 in addition to the fan filter units 14 and 15 and the pressure sensor 22. The control device 80 is a device that controls the air supply fan 14a and the air returning fan 15a. Although not shown, the control device 80 is implemented by an electronic circuit including a central processing unit (CPU), a read only memory (ROM), a random access memory (RAM), various interfaces, and the like. A program stored in the ROM is read and loaded into the RAM, and the CPU executes various kinds of processing.

In the example shown in FIG. 3, an input side of the control device 80 is connected to the pressure sensor 22 via a wire, and an output side thereof is connected to the air supply fan 14a and the air returning fan 15a via a wire. The control device 80 controls the air supply fan 14a and the air returning fan 15a in a predetermined manner based on a detection value of the pressure sensor 22 such that the room pressure of the room R6 (see FIG. 2) is predetermined set pressure. One of the air supply fan 14a and the air returning fan 15a may rotate at a constant speed. Although FIG. 3 shows a configuration related to air supply and returning in the room R6 (see FIG. 2), one control device may control air supply fans and air returning fans of a plurality of clean rooms (the processing room R3 and the rooms R6, R7, and R8: see FIG. 2).

### <Comparative Example>

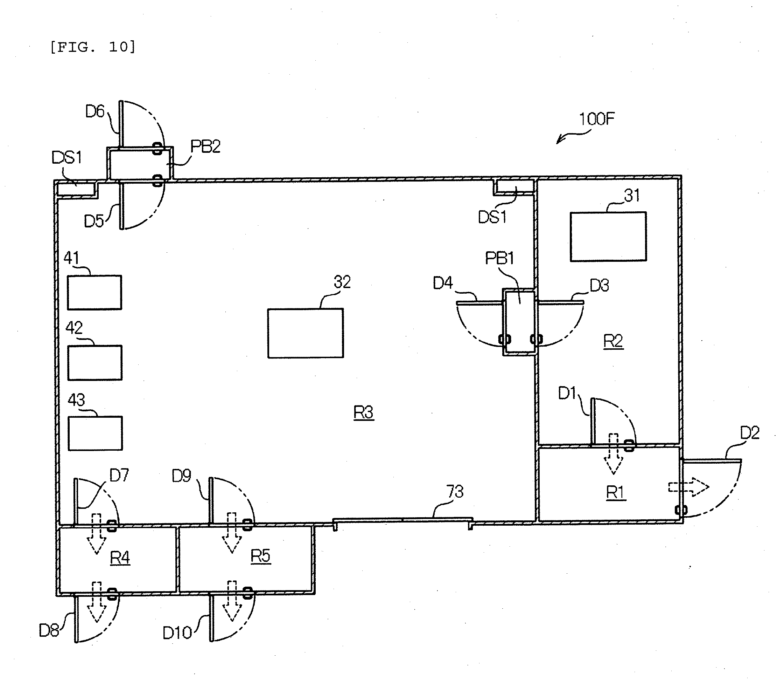

FIG. 10 is a diagram showing a layout of rooms of a clean room facility 100F according to a comparative example.

In the comparative example shown in FIG. 10, the three incubators 41 to 43 are provided in the processing room R3, and the rooms R6, R7, and R8 (see FIG. 1) for accommodating the incubators 41 to 43 are not provided. Since aerosol may adhere to equipment or a floor surface (sample contamination may occur) in a process in which an operator takes in and out cells or the like in the incubators 41 to 43, there are few cases where treatment is performed in a state in which cells or the like of a plurality of patients are mixed in one processing room R3. That is, in the clean room facility 100F, an operator treats cells and the like of one patient in the processing room R3, then the processing room R3 is sterilized (or wiped with alcohol), and then cells and the like of another patient are handled.

Due to such circumstances, treatment of cells or the like of another patient is in a waiting state until treatment of cells or the like of the one patient is completed, and it is difficult to increase work efficiency. It is possible to provide the incubators 41 to 43 with a high-temperature sterilization function, but in this case, costs of the incubators 41 to 43 increase.

In the first embodiment, the incubators 41 to 43 are individually accommodated in the three rooms R6, R7, and R8 as shown in FIG. 1. Therefore, for example, even when sample contamination occurs in the room R6, the sample contamination can be prevented from spreading to the processing room R3 and the other rooms R7 and R8. Since a range to be sterilized (for example, the room R6) is small, labor and costs required for sterilization can be reduced.

### <Treatment During General Use>

During general use of the clean room facility 100, the air conditioner 9 and the fan filter units 11 to 19 shown in FIG. 2 are driven in a predetermined manner. The first damper 56a is in an open state during general use of the room R6 corresponding to the first damper 56a, and the chamber C3 and the room chamber C6 communicate with each other. Accordingly, clean air is supplied from the chamber C3 to the room R6 sequentially through the first damper 56a and the room chamber C6.

The second damper 56b is in an open state during general use of the room R6 corresponding to the second damper 56b, and the duct shaft DS6 and the chamber C3 communicate with each other. Accordingly, air is returned from the room R6 to the chamber C3 sequentially through the duct shaft DS6 and the second damper 56b. The air is cleaned in a process of passing through the fan filter units 11 and 12 on the air supply side and the filter 9a of the air conditioner 9.

For example, an operator can treat cells or the like of a patient in the incubator 41 (see FIG. 1) of the room R6, and another operator can treat cells or the like of another patient in the incubator 42 (see FIG. 1) of the room R7, and thus work efficiency is increased.

### <Treatment During Sterilization>

For example, an operator may spill a sample when the operator works in the room R8. After cells or the like of a patient are treated as a sample, sterilization is often performed when cells or the like of another patient are treated (during a so-called changeover).

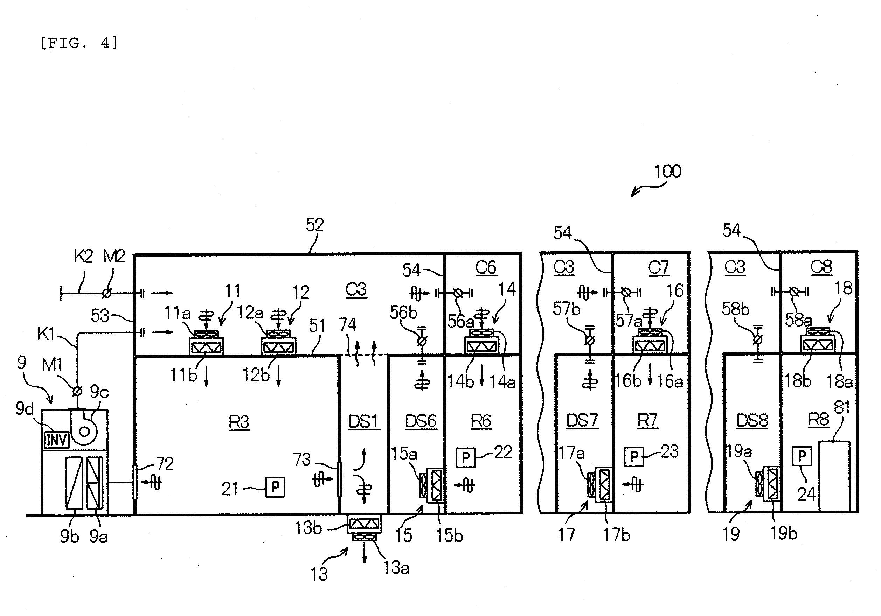

FIG. 4 is a diagram showing an example related to treatment during sterilization of the room R8.

For example, when the room R8 is sterilized, a decontamination device 81 that injects a predetermined sterilizing gas (a hydrogen peroxide gas or the like) is placed in the room R8. when an operation switch (not shown) of the decontamination device 81 is pressed in a state where a door 8b (see FIG. 1) of the room R8 is closed, the sterilizing gas is injected from the decontamination device 81, and the room R8 is filled with the sterilizing gas. As described above, when sterilization of at least one of the plurality of rooms R6, R7, and R8 (for example, the room R8) is performed, the room is filled with a predetermined sterilizing gas.

During sterilization of the room R8, an air supply fan 18a for supplying air to the room R8 is stopped, and an air returning fan 19a for returning air from the room R8 is also stopped. As described above, the room R8 is partitioned from the processing room R3 and the other rooms R6 and R7, and the door 6b is closed and the room R8 is in a sealed state. Since a volume and a surface area of the room R8 are relatively small, sterilization can be completed in a short time.

A first damper 58a is in a closed state during sterilization of the room R8 corresponding to the first damper 58a, and blocks communication between the chamber C3 and the room chamber C8. A second damper 58b is in a closed state during sterilization of the room R8 corresponding to the second damper 58b, and blocks communication between a duct shaft DS8 and the chamber C3. Accordingly, it is possible to prevent the sterilizing gas from flowing from the room R8 into the processing room R3 sequentially through the duct shaft DS8 and the chamber C3 during sterilization of the room R8.

During sterilization of the predetermined room R8, the air supply fan 18a and the air returning fan 19a corresponding to the room R8 may be stopped, and air supply fans 14a and 16a and air returning fans 15a and 17a corresponding to the other rooms R6 and R7 may be driven. In addition, the air conditioner 9 and the fan filter units 11 to 13 of the processing room R3 may be driven during sterilization of the room R8. Accordingly, the other rooms R6 and R7 and the processing room R3 can be used during sterilization of the room R8, and thus work efficiency during treating a sample can be improved.

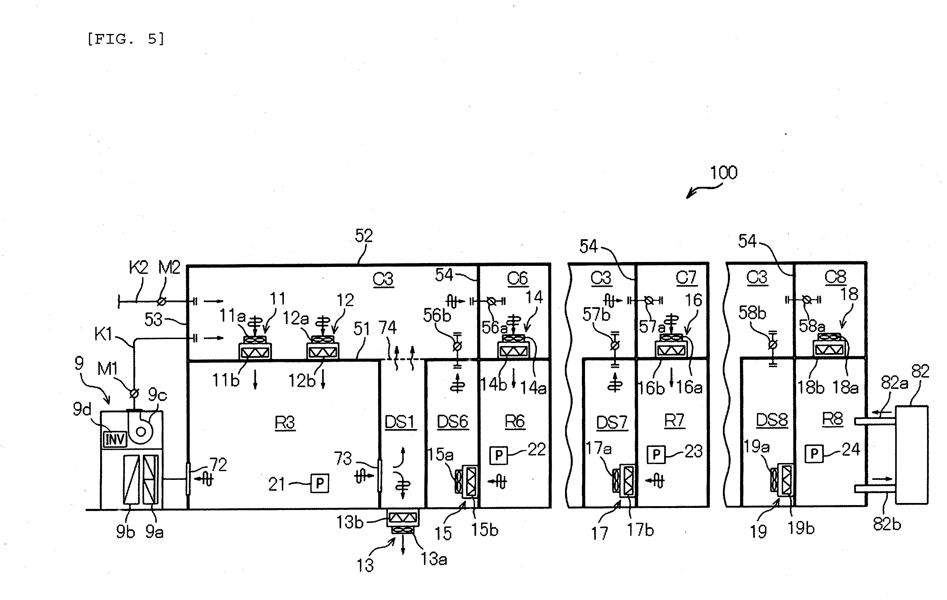

FIG. 5 is a diagram showing another example related to treatment during sterilization of the room R8.

A decontamination device 82 shown in FIG. 5 is a device that fills a predetermined sterilizing gas (a hydrogen peroxide gas or the like) into the room R8 to be sterilized and then fills a predetermined catalyst gas (for example, a catalyst containing platinum or palladium) for detoxifying the sterilizing gas. An air supply side hose 82a and an exhaust side hose 82b are connected to the decontamination device 82. The air supply side hose 82a is a pipe that guides a predetermined sterilizing gas or catalyst gas from the decontamination device 82 to the room R8. The exhaust side hose 82b is a pipe that guides an exhaust gas from the room R8 to the decontamination device 82.

When the room R8 is sterilized, the decontamination device 82 supplies a predetermined sterilizing gas to the room R8 via the air supply side hose 82a, and exhausts the gas from the room R8 via the exhaust side hose 82b. After the room R8 is sterilized, the decontamination device 82 supplies a predetermined catalyst gas for detoxifying the sterilizing gas to the room R8 via the air supply side hose 82a and exhausts the gas from the room R8 via the exhaust side hose 82b. The same treatment is performed when the other rooms R6 and R7 are sterilized.

A predetermined catalyst filter (not shown) for detoxifying the sterilizing gas may be incorporated in the decontamination device 82. A decomposition device (not shown) including a catalyst filter (not shown) may be separately provided.

### <Effects>

According to the first embodiment, since the rooms R6, R7, and R8 in which the incubators 41 to 43 (see FIG. 1) are arranged are individually provided, it is possible to perform work in parallel in the three rooms R6, R7, and R8 in addition to the processing room R3. Accordingly, work efficiency in handling a sample is increased. When an operator sterilizes the room R8 after the operator treats a predetermined sample in the room R8, it is not particularly necessary to sterilize the processing room R3 and the other rooms R6 and R7. Therefore, time and costs required for sterilization can be reduced.

According to the first embodiment, a structure of the clean room facility 100 is not particularly complicated, and an operator can efficiently treat a sample with a relatively simple structure. Since it is not particularly necessary to provide the incubators 41 to 43 with a sterilization function, cost reduction can be achieved. When an operator handles cells or the like of a patient in the room R6, another operator can handle cells or the like of another patient in the room R7. In this manner, according to the first embodiment, it is possible to provide the clean room facility 100 that is easy to use and improve efficiency of sample preparation.

### <<Second Embodiment>>

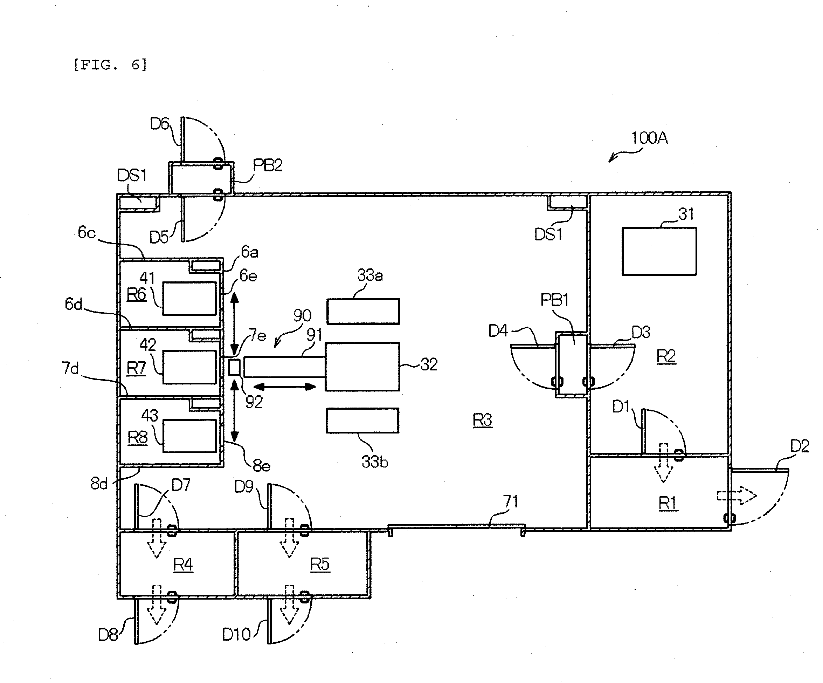

The second embodiment is different from the first embodiment in that a shutter 6e (see FIG. 6) is provided in the room R6, and shutters 7e and 8e (see FIG. 6) are provided in the rooms R7 and R8. The second embodiment is different from the first embodiment in that a conveyance machine 90 (see FIG. 6) is used to move a sample. Other configurations are the same as those in the first embodiment. Therefore, portions different from those in the first embodiment will be described, and description of overlapping portions will be omitted.

FIG. 6 is a diagram showing a layout of rooms of a clean room facility 100A according to the second embodiment.

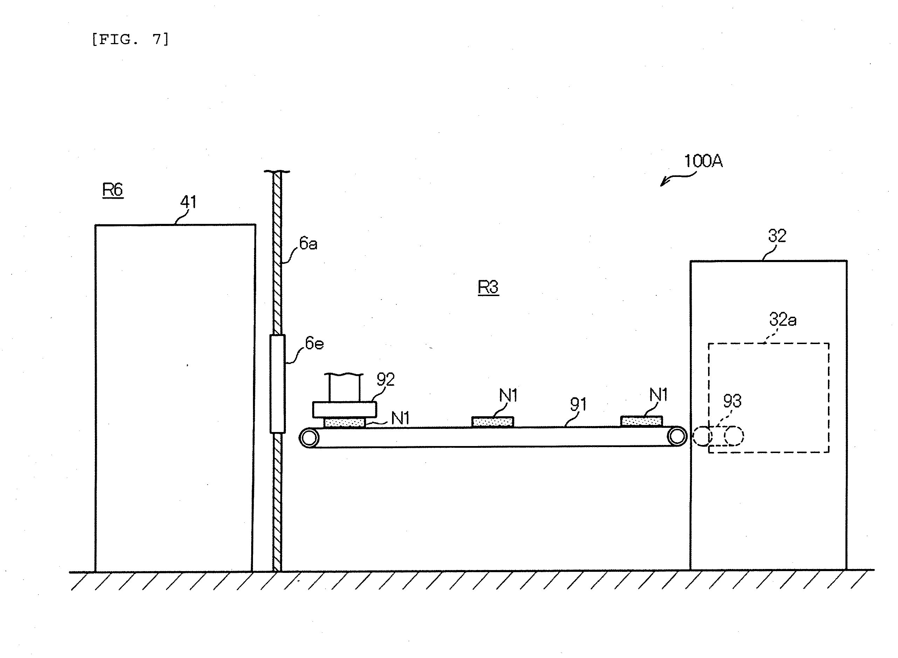

An arrow near the conveyance machine 90 in FIG. 6 indicates a direction in which a container N1 (see FIG. 7) of a sample moves. The openable and closable shutter 6e is provided in the room R6 of the clean room facility 100A shown in FIG. 6. The shutter 6e is an openable and closable window used for moving a sample, and is provided in the partition 6a of the room R6. Similarly, the shutters 7e and 8e are also provided in the rooms R7 and R8. For example, when the container N1 (see FIG. 7) of a sample is transferred between the conveyance machine 90 and the incubator 41, the shutter 6e of the room R6 is automatically opened.

A size of each of the shutters 6e, 7e, and 8e (windows) is preferably a size at which a person cannot enter and exit. For example, it is preferable that each of the shutters 6e, 7e, and 8e (windows) has a rectangular opening, a length in a lateral direction is 40 cm or less, and a length in a height direction is 40 cm or less. By making dimensions of the shutters 6e, 7e, and 8e relatively small in this manner, it is possible to prevent a room pressure fluctuation in the processing room R3 accompanying with opening and closing of the shutters 6e, 7e, and 8e, and prevent sample contamination in the processing room R3.

As shown in FIG. 6, the clean room facility 100A includes the conveyance machine 90. The conveyance machine 90 is a machine that conveys the container N1 (see FIG. 7) of a sample. That is, the conveyance machine 90 has a function of conveying the container N1 (see FIG. 7) of a sample between the safety cabinet 32 (the first device) and the incubators 41 to 43 (the second devices) . In the example shown in FIG. 6, the conveyance machine 90 includes a belt conveyor 91, a sorting robot 92, and a belt conveyor 93 (see FIG. 7) provided inside the safety cabinet 32. Automated warehouses 33a and 33b of consumable equipment or the like are provided on both sides of the safety cabinet 32.

FIG. 7 is a diagram including the conveyance machine 90 of the clean room facility 100A.

In the following description, a case where the container N1 of a sample is conveyed from the safety cabinet 32 to the incubator 41 will be described as an example. It is also possible to move the container N1 of a cultured sample from any one of the incubators 41 to 43 to the safety cabinet 32.

The belt conveyor 93 (see FIG. 7) shown in FIG. 7 is a device that moves the container N1 of a sample treated in the safety cabinet 32 to the belt conveyor 91 on a downstream side. In the example shown in FIG. 7, the belt conveyor 93 is provided in a work area 32a of the safety cabinet 32. The belt conveyor 91 conveys the container N1 conveyed from the belt conveyor 93 on an upstream side to the vicinity of the sorting robot 92. The sorting robot 92 grips (or aspirations) the container N1 conveyed by the belt conveyor 91, and accommodates the container N1 in one of shelves (not shown) of the incubators 41 to 43.

When the container N1 is conveyed by the conveyance machine 90, an incubator (for example, the incubator 41) that is a conveyance destination is specified in association with identification information of the container N1. A shutter (not shown) may be provided in a housing (not shown) surrounding a movement region of the sorting robot 92, and the sorting robot 92 may sort the container N1 received through the shutter to any one of the incubators 41 to 43.

### <Effects>

According to the second embodiment, since the conveyance machine 90 is used to move the container N1 of a sample, it is possible to reduce time and effort when moving the container N1. Since the number of persons who work in the processing room R3 or the like can be reduced, sample contamination such as spilling of a sample on a floor is less likely to occur. Accordingly, since a frequency of sterilization of the processing room R3 and the rooms R6 to R8 is reduced, costs can be reduced.

### <<Modification>>

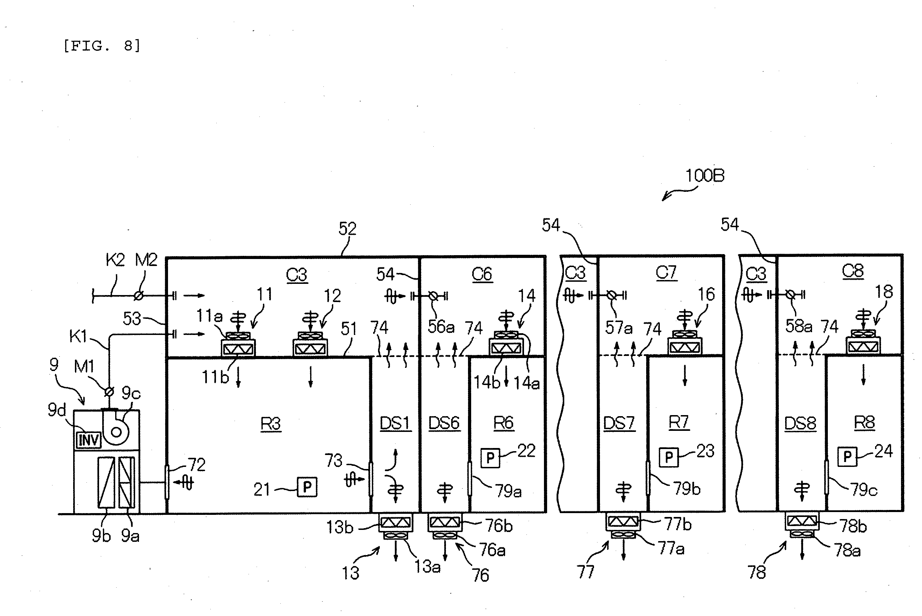

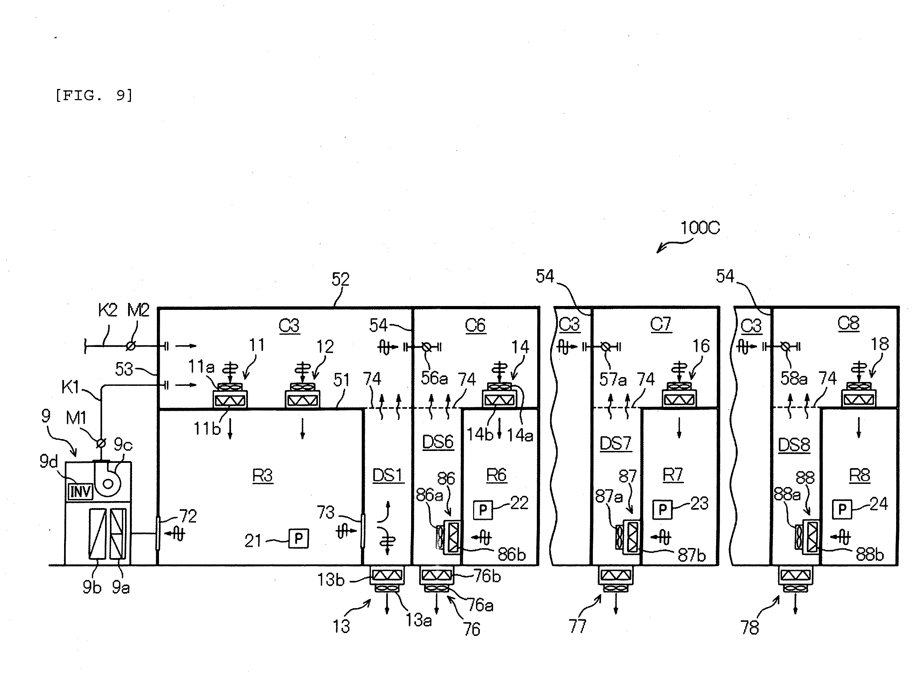

Although the clean room facilities 100 and 100A according to the invention are described in the embodiments, the invention is not limited thereto, and various modifications can be made. For example, although a configuration in which air is guided from the room R6 (see FIG. 2) to the processing room R3 through the duct shaft DS6 is described in the embodiments, the invention is not limited thereto, and for example, the configuration may be modified as in modifications shown in FIG. 8 and FIG. 9.

FIG. 8 is a schematic cross-sectional view showing a clean room facility 100B according to a first modification.

For example, the clean room facility 100B shown in FIG. 8 has a configuration corresponding to the room R6, and includes the room chamber C6, fan filter units 14 and 76, the duct shaft DS6, and the first damper 56a. The room chamber C6 is a space behind a ceiling of the room R6. The room chamber C6 is partitioned from the adjacent room chamber C7 by a wall member (not shown), and is partitioned from the processing room R3 by the separation wall 54. The separation wall 54 of the room chamber C6 is provided with the first damper 56a. During general use of the room R6, the first damper 56a is in an open state.

The air supply fan 14a of the fan filter unit 14 is a blower that supplies air to the room R6, and is provided in the room chamber C6. An air returning fan 76a (a first air returning fan) of the fan filter unit 76 has a function of returning at least a part of air flowing out from the room R6 to the room chamber C6 through the duct shaft DS6. That is, the air returning fan 76a (the first air returning fan) has a function of guiding a part of the air flowing out from the room R6 via a predetermined gap 79a to the duct shaft DS6 and exhausting remaining air. The porous plate 74 (or grating) may be provided at a downstream end of the duct shaft DS6. The same applies to configurations corresponding to the other rooms R7 and R8.

In other words, the first modification shown in FIG. 8 is different from the first embodiment (see FIG. 2) in that, for example, a part of air in the room R6 is returned to the room chamber C6 through the duct shaft DS6. According to such a configuration, since air hardly moves from the room R6 to the processing room R3, it is possible to reliably prevent aerosol from flowing into the processing room R3 immediately after sample contamination occurs in the room R6.

FIG. 9 is a schematic cross-sectional view showing a clean room facility 100C according to a second modification.

In the second modification shown in FIG. 9, fan filter units 86, 87, and 88 are added to the first modification (see FIG. 8). For example, the fan filter unit 86 corresponding to the room R6 includes an air returning fan 86a (a second air returning fan) and a filter 86b. The air returning fan 86a has a function of returning at least a part of air flowing out from the room R6 to the room chamber C6 through the duct shaft DS6. That is, the air returning fan 86a (the second air returning fan) is a blower that guides the air flowing out from the room R6 to the duct shaft DS6 via the air returning fan 86a. The same applies to configurations corresponding to the other rooms R7 and R8. According to such a configuration, for example, a flow rate of air flowing from the room R6 through the duct shaft DS6 can be appropriately adjusted by driving the air returning fans 76a and 86a together.

Although a case where the processing room R3 and the rooms R6, R7, and R8 are positive pressure rooms is described in the embodiments, the processing room R3 and the rooms R6, R7, and R8 may be negative pressure rooms depending on an application.

Although a case where room pressure of the processing room R3 is different from room pressure of the rooms R6, R7, and R8 is described in the embodiments, the invention is not limited thereto. That is, the room pressure of the processing room R3 may be equal to the room pressure of the rooms R6, R7, and R8.

Although a configuration in which no duct is particularly provided in the chamber C3 or the room chambers C6, C7, and C8 is described in the embodiments, the invention is not limited thereto. That is, a duct (not shown) that guides air supplied through the ducts K1 and K2 (see FIG. 2) to each fan filter unit on an air supply side may be separately provided.

Although a configuration in which the three rooms R6, R7, and R8 are provided adjacent to the processing room R3 in the embodiments, the number or an arrangement of the rooms may be changed as appropriate.

Although the rooms R6, R7, and R8 may be provided at the time of constructing the clean room facility 100, the rooms R6, R7, and R8 may be installed later. In this case, the processing room R3 may be provided in advance with a partition having a window or a door (not shown) that is not connected to a room, and the room may be added to the processing room R3 later.

Although a case where the "second devices" arranged in the rooms R6, R7, and R8 are the incubators 41 to 43 (see FIG. 1) is described in the embodiments, the invention is not limited thereto. That is, in addition to an imaging device and an inspection device, a sub-culturing device, a seeding device, a cell collection device, and the like may be arranged in the rooms R6, R7, and R8 as the "second devices" related to culture or inspection of a sample. For example, the incubator 41 may be provided in the room R6, the imaging device may be provided in another room R7, and the inspection device may be provided in the remaining room R8. That is, the "second devices" provided in the rooms R6, R7, and R8 may be of different types. A plurality of "second devices" may be provided in one room (for example, the room R6) .

Although a configuration in which the air returning fan 15a is installed to return air flowing out from the room R6 (see FIG. 2) to the chamber C3 behind the ceiling of the processing room R3 through the duct shaft DS6 is described in the first embodiment, the invention is not limited thereto. For example, an air returning fan (not shown) may be installed to return at least a part of the air flowing out from the room R6 (see FIG. 2) to the chamber C3 behind the ceiling of the processing room R3 through the duct shaft DS6 and exhaust the remaining air. The same applies to the air returning fans 17a and 19a in the other rooms R7 and R8.

Although a configuration in which the conveyance machine 90 (see FIG. 7) includes the belt conveyors 91 and 93 and the sorting robot 92 is described in the second embodiment, the invention is not limited thereto. For example, one or more of the belt conveyors 91 and 93 and the sorting robot 92 may be omitted, and a human hand may be involved in moving the container N1.

The embodiments and the first to third modifications can be combined as appropriate. For example, the second embodiment (a configuration including the conveyance machine 90: see FIG. 7) and the first modification (see FIG. 8) may be combined. In addition, various combinations such as a combination of the second embodiment (see FIG. 7) and the second modification (see FIG. 9) can be made.

Although a case where the clean room facilities 100 and 100A are used for preparing cells or the like is described in the embodiments, the invention is not limited thereto. That is, the embodiments can be applied to various other fields such as manufacture of a semiconductor and a precision machine and a food industry in addition to manufacture of a pharmaceutical product.

Further, the embodiments described above have been described in detail to describe the invention in an easy-to-understand manner, and the invention is not necessarily limited to those including all the configurations described above. In addition, another configuration can be added to, deleted from, or replaced with a part of configurations of each embodiment.

Mechanisms and configurations described above indicate what is considered to be necessary for explanation, and not all mechanisms and configurations are necessarily shown on a product.

### Reference Signs List

100, 100A, 100B, 100C, 100D: clean room facility
32: safety cabinet (first device)
41, 42, 43: incubator (second device)
6a, 7a, 8a: partition
6b, 7b, 8b: door
6d, 7d: side wall (wall)
6e, 7e, 8e: shutter (window)
14a, 16a, 18a: air supply fan
15a, 17a, 19a: air returning fan
54: separation wall
56a, 57a, 58a: first damper
56b, 57b, 58b: second damper
76a, 77a, 78a: air returning fan (first air returning fan)
86a, 87a, 88a: air returning fan (second air returning fan)
79a, 79b, 79c: gap
90: conveyance machine
C3: chamber
C6, C7, C8: room chamber
DS6, DS7, DS8: duct shaft
N1: container
R3: processing room
R6, R7, R8: room

## Claims

1. A clean room facility comprising:
a processing room configured to allow a first device used for preparing a sample to be arranged therein;
a plurality of rooms configured to allow second devices related to culture or inspection of the sample to be arranged therein;
a partition partitioning the processing room and each of the rooms;
an openable and closable door or window provided in the partition and used for moving of the sample; and
a wall partitioning adjacent rooms among the plurality of rooms.

2. The clean room facility according to claim 1, wherein
when sterilization of at least one of the plurality of rooms is performed, the room is filled with a predetermined sterilizing gas.

3. The clean room facility according to claim 1, wherein
cleanliness of the processing room is equal to cleanliness of each of the plurality of rooms, and
room pressure of the processing room is different from room pressure of each of the plurality of rooms.

4. The clean room facility according to claim 3, wherein
the room pressure of the processing room is higher than the room pressure of each of the plurality of rooms.

5. The clean room facility according to claim 1, further comprising:
a conveyance machine configured to convey a container of the sample, wherein
the conveyance machine conveys the container between the first device and the second device provided in a predetermined room.

6. The clean room facility according to claim 1, further comprising:
a room chamber provided behind a ceiling of each of the plurality of rooms;
an air supply fan provided in the room chamber and configured to supply air to the room corresponding to the room chamber; and
an air returning fan configured to return at least a part of air flowing out from the room to a chamber behind a ceiling of the processing room through a duct shaft, wherein
the room chamber is partitioned from the adjacent room chamber by a wall member, and is partitioned from the processing room by a separation wall.

7. The clean room facility according to claim 6, further comprising:
a first damper provided in the separation wall in a manner of corresponding to each of the rooms and configured to enable or block communication between the chamber and the room chamber; and
a second damper provided in a vicinity of a downstream end of the duct shaft in a manner of corresponding to each of the rooms and configured to switch to enable or block communication between the duct shaft and the chamber.

8. The clean room facility according to claim 7, wherein
the first damper enables communication between the chamber and the room chamber during general use of the room corresponding to the first damper, and
the second damper enables communication between the duct shaft and the chamber during general use of the room corresponding to the second damper.

9. The clean room facility according to claim 7, wherein
the first damper blocks communication between the chamber and the room chamber during sterilization of the room corresponding to the first damper, and
the second damper blocks communication between the duct shaft and the chamber during sterilization of the room corresponding to the second damper.

10. The clean room facility according to claim 6, wherein
during sterilization of a predetermined room among the plurality of rooms, the air supply fan and the air returning fan corresponding to the room are stopped, and the air supply fans and the air returning fans corresponding to the other rooms are driven.

11. The clean room facility according to claim 1, further comprising:
a room chamber provided behind a ceiling of each of the plurality of rooms;
an air supply fan provided in the room chamber and configured to supply air to the room corresponding to the room chamber; and
an air returning fan configured to return at least a part of air flowing out from the room to the room chamber corresponding to the room through a duct shaft, wherein
the room chamber is partitioned from the adjacent room chamber by a wall member, and is partitioned from the processing room by a separation wall.

12. The clean room facility according to claim 11, further comprising:
a first air returning fan, as the air returning fan, configured to guide a part of air flowing out from the room through a predetermined gap to the duct shaft and exhaust remaining air.

13. The clean room facility according to claim 12, further comprising:
a second air returning fan, as the air returning fan, configured to guide air flowing out from the room to the duct shaft via the second air returning fan.
